Europäisches Patentamt

European Patent Office

Office européen des brevets

(11) Numéro de publication: **0 148 075**
A2

## (12) DEMANDE DE BREVET EUROPEEN

(21) Numéro de dépôt: 84402658.3

(22) Date de dépôt: 19.12.84

(51) Int. Cl.⁴: **C 12 P 21/00**
A 61 K 39/395, C 12 N 5/00
G 01 N 33/577

(30) Priorité: 19.12.83 FR 8320298

(43) Date de publication de la demande:
10.07.85 Bulletin 85/28

(84) Etats contractants désignés:
AT BE CH DE FR GB IT LI LU NL SE

(71) Demandeur: INSTITUT PASTEUR
25-28, rue du Docteur Roux
F-75724 Paris Cedex 15(FR)

(71) Demandeur: INSTITUT NATIONAL DE LA SANTE ET DE
LA RECHERCHE MEDICALE (INSERM)
101, rue de Tolbiac
F-75654 Paris Cedex 13(FR)

(71) Demandeur: Etablissement Public dit: CENTRE
NATIONAL DE LA RECHERCHE SCIENTIFIQUE (CNRS)
15, Quai Anatole France
F-75007 Paris(FR)

(72) Inventeur: Dray, Fernand
4, rue Guynemer
F-75006 Paris(FR)

(72) Inventeur: Somme, Gérard
35, rue des Morillons
F-75015 Paris(FR)

(72) Inventeur: Theze, Jacques
74, rue Desnouettes
F-75015 Paris(FR)

(72) Inventeur: David, François
69bis, rue Henri-Barbusse
F-93260 Les Lilas(FR)

(74) Mandataire: Phélip, Bruno et al,
c/o Cabinet Harlé & Phélip 21, rue de la Rochefoucauld
F-75009 Paris(FR)

(54) Anticorps monoclonaux anti-PG E spécifiques, leur préparation et leurs applications biologiques.

(57) On décrit la préparation d'hybridomes sécréteurs d'anticorps monoclonaux anti-prostaglandines monospécifiques et de haute affinité, notamment des anticorps anti-PGE et plus particulièrement anti-PG $E_2$. Ces anticorps sont utilisables comme réactifs dans des procédés immunologiques de dosage sélectif des prostaglandines ou comme agents thérapeutiques.

EP 0 148 075 A2

Croydon Printing Company Ltd.

Anticorps monoclonaux anti-PG E spécifiques, leur préparation et leurs applications biologiques

L'invention concerne des anticorps monoclonaux spécifiques dirigés contre une PG E, telle que PG $E_1$ ou PG $E_2$, le procédé de préparation de ces anticorps et des hybridomes qui les sécrètent ainsi que l'application biologique de ces anticorps.

La préparation de lignées d'hybridomes, obtenus par fusion de cellules de myélome de souris et de lymphocytes de souris immunisées contre un antigène donné a été largement pratiquée depuis qu'en 1975, KOHLER et MILSTEIN ont décrit une technique dans Nature 256 495-497. Par exemple, la demande FR-A-2519650 de SORIANO et coll décrit l'obtention d'hybridomes par fusion de splénocytes de souris immunisées contre le virus de l'Herpès simplex et de cellules de myélome murin ainsi que leur clonage et l'isolement des anticorps qu'ils secrètent; EP-A- 0 044 441 décrit l'obtention d'hybrodomes secréteurs d'anticorps anti-haptènes, comme les antibiotiques, par une technique analogue utilisant des animaux immunisés par l'haptène couplé chimiquement à un agent immunogénique. On sait toutefois qu'il n'est pas possible d'appliquer, sans l'adapter, une technique donnée, à la production de n'importe quel hybridome et anticorps monoclonal correspondant. Les conditions d'immunisation des animaux, de culture des cellules sont fonction de l'antigène de départ et doivent être adaptées à chaque cas particulier, pour conduire à l'obtention avec de bons rendements, d'anticorps monoclonaux spécifiques et de haute affinité pour l'antigène. Dans le cas des prostaglandines, la question de l'affinité est cruciale :

7683 EX
MJB

2

du fait de la concentration habituellement faible de ces composés par rapport à leur environnement lipidique, il faut un système de révélation particulièrement sensible et par conséquent des anticorps de très haute affinité.

Des anticorps polyclonaux dirigés contre l'haptène PG $E_2$ ont été obtenus dans diverses espèces telles que, lapin, chèvre, mouton par immunisation après leur couplage à différentes protéines mais dans tous les cas les anticorps obtenus ne sont pas monospécifiques et en général en quantité minoritaire.

On sait que la molécule de PG $E_2$ peut être modifiée lors de son couplage chimique à une protéine et in vivo sous l'action d'enzymes, telles que déhydrase et isomérases; parmi les produits alors formés la PG $B_2$ est majoritaire, ce qui entraîne la présence d'une majorité d'anticorps anti PG $B_2$ dans les immunosérums obtenus par immunisation d'animaux avec la PG $E_2$. Une modification du noyau prostanoïque se produit aussi avec d'autres prostaglandines, comme les PG E et PG D, qui ont dans leurs molécules un groupe $\beta$-hydroxycéto ou comme les PG A, qui ont une fonction cétone conjuguée.

De ce fait, il était souhaitable pour obtenir des anticorps mono spécifiques dirigés contre l'une de ces prostaglandines d'utiliser une technique autre que celle classique d'immunisation in vivo.

Par ailleurs des anticorps polyclonaux spécifiques de la PG $E_1$ ou de la PG $E_3$ n'avaient jamais été isolés.

Dans la demande de brevet DE-A-3218269, il est décrit un procédé d'obtention d'anticorps

3

monoclonaux anti-PG F et l'utilisation de ceux-ci dans des procédés de dosage radio-immunologiques desdites prostaglandines F. La molécule de PG F ne subit pas d'altération lors de son couplage à une protéine ou in vivo par voie enzymatique et l'obtention d'anticorps monoclonaux anti-PG F ne nécessitait aucune adaptation particulière de la méthode classique utilisant l'hybridation cellulaire, ce qui ne pouvait s'appliquer aux prostaglandines précédemment citées à noyaux prostanoïque fragile.

La présente invention concerne les prostaglandines dont le noyau prostanoïque comporte un groupe $\beta$-hydroxycéto ou une fonction cétone conjuguée, plus particulièrement les PGE et tout spécialement la PG $E_2$.

La présente invention permet notamment la préparation de quantités importantes d'anticorps monoclonaux dirigés contre chacune des PG E, monospécifiques et de haute affinité.

De tels anticorps seront avantageusement utilisés à la place des anticorps polyclonaux dans les procédés de dosage immunologiques; leur forte affinité et grande spécificité permettant des dosages sélectifs et sensibles notamment dans des milieux où les prostaglandines sont en mélange. En outre, le procédé selon l'invention permettant l'obtention de grandes quantités d'anticorps purs, leur emploi comme réactifs biologiques et pour des traitements thérapeutiques devient possible; on a en effet notamment montré que la PG $E_2$ est impliquée in vivo dans de nombreux processus secrétoires et dans le déclanchement des syndromes immunodéficitaires. On pourra se référer notamment aux articles de Goodwin et col. dans Journal of Clinical Immunology 3 (4), p. 295 (1983) et New England Journal of Medicine 297 (18) p 963-968 (1977).

Selon l'invention, on prépare des hybridomes secréteurs de ces anticorps monoclonaux en effectuant les opérations suivantes :

a) immunisation de souris par l'une des prostaglandines PG E couplée à une protéine immunogénique, telle que le sérum albumine ou la tyroglobuline, à l'aide d'un agent de couplage tel qu'un carbodiimide,

b) isolement des cellules spléniques des animaux immunisés,

c) fusion des cellules de rate avec des cellules d'un myélome de souris, en présence d'un agent de fusion tel que le polyéthylène glycol ou le virus de Sendaï,

d) sélection des hybridomes par culture des cellules ayant subi l'étape de fusion sur un milieu approprié, c'est-à-dire où les cellules non fusionnées ne se développent pas, et

e) isolement de clones et sous-clones des hybridomes secréteurs d'un anticorps, par culture soit en milieu solide soit par dilution limite en milieu liquide.

Pour l'immunisation, dans une forme préférée de réalisation de l'invention, le PG E est couplée à la tyroglobuline par l'éthyl-1 (diméthylamino-3-propyl)-3 carbodiimide, selon une méthode décrite par DRAY F. et coll. dans "Methods in Enzymology 86 258 (1982)- Ed$^{ns}$ W.F.M. Lands et W.L. Smith-Academic Press, N.Y.".

On a constaté que par cette méthode moins de 10% de la PG $E_2$ était transformée en PG $B_2$ lors du couplage, ce qui est un autre avantage de l'invention. On effectue au moins deux injections successives du complexe PG E/protéine pour immuniser les animaux:

- la première injection est, pour une souris de 15 à 20g, de 5 à 20 µg d'équivalent PG E c'est-à-dire de PG E sous forme de complexe, en émulsion dans de l'adjuvant complet de Freund ou en présence d'un autre adjuvant classiquement utilisé en immunologie,

- la seconde injection de la même émulsion a lieu environ un mois plus tard, mais avec une dose de 5 à 10 µg d'équivalent PG E,

- une injection supplémentaire, trois jours avant le prélèvement de la rate de l'animal et qui comprend 1 à 5 µg d'équivalent de PG E en solution dans un tampon physiologique, peut être pratiquée en particulier pour la PG $E_2$.

On entend ici par µg d'équivalent PG E le poids de la PG E contenue dans le complexe immunogénique PG E/protéine injecté.

La répétition des injections du complexe est un aspect de l'invention particulièrement important dans le cas de la PG $E_2$, après une seule injection les anticorps sont dirigés presque uniquement contre la PG $B_2$.

Lorsque les animaux sécrètent suffisamment d'anti-PG E, leurs splénocytes sont isolés selon les techniques habituelles. La fusion de ceux-ci avec les cellules de myélome est réalisée en appliquant la méthode de KOHLER et MILSTEIN modifiée, décrites par GERHARD W. " Monoclonal antibodies-(1980) p. 370 Ed[S] Kenneth, H., Mc Kearn T.S. et Bechtol K.B. - Plenum Press". On utilise comme agents de fusion le virus de Sendaï ou de préférence un polyéthylène glycol et notamment le

6

PEG 1000 commercialisé par MERCK. Par ailleurs les cellules peuvent être celles du myélome SP2/O-Ag décrites par SCHULMANN et coll. dans : Nature 276 p. 269 (1978) ou les cellules du myélome X 63 Ag 8/653 décrites par KEARNEY J.F. et coll. dans J. Immunol. 123 p. 1548 (1979) ou encore les cellules du myélome $P_3$-NS 1/1 Ag 4-1 décrite par KOHLER G. et coll. dans Eur. J. Immunol. 6 292 (1976). La sélection des hybridomes selon l'invention est effectuée dans un milieu où les différentes cellules de départ ne peuvent se développer. C'est habituellement le milieu RPMI-1640 (commercialisé par GIBCO-USA) qui est utilisé complémenté avec du sérum animal, des acides aminés, du pyruvate de sodium, un mélange d'antibiotiques et d'antifongiques et des agents de sélection tels que l'hypoxanthine et l'azasérine. On obtient une quantité convenable d'hybridomes en 5 à 30 jours. Seules les cultures qui ont une activité anti-PG E sont clonées et sous-clonées après avoir été mises en évidence en effectuant un test radioimmunologique, comme décrit dans ce qui suit. Les clonages et sous-clonages sont réalisés selon une technique connue sur une microplaque en introduisant une cellule pour 3 puits; chaque puits contient, 200 µl d'un milieu de culture approprié (par exemple celui utilisé pourla sélection, dans lequel les agents de sélection peuvent être omis) et des thymocytes de souris à raison de $2 \times 10^5$ par puits.

Pour mettre en évidence les clones secréteurs d'anticorps anti-PG E, on effectue un dosage radioimmunologique, basé sur un essai de liaison, pour les surnageants de chaque puits où un clone s'est développé. Pour réaliser cet essai de liaison radioimmunologique, on prélève 100 ul de surnageant dans un puits et on le fait incuber 12 heures à 4°C avec 10 pg de la PG E du type concerné, marquée au tritium et d'activité spécifique environ 160 Ci/mM (commercialisé par Amersham) en solution dans 200 ul d'un mélange tampon phosphate et gélétine; On ajoute alors 1 ml de polyéthylèneglycol 6000 (commercialisé par Merck)en solution aqueuse à 30%, ce qui précipite le complexe PG E tritiée/anticorps, et on centrifuge la suspension avant d'éliminer le surnageant. Le culot est repris dans 5 ml de liquide pour scintigraphie (commercialisé par New England Nuclear, sous la marque AQUASOL), et on effectue une mesure de la radioactivité de cet échantillon avec un compteur $\beta$ à scintillation liquide;

Deux lignées d'hybridomes sécréteurs anti-PG $E_2$ et une lignée d'hybridomes anti-PG $E_1$, aux propriétés particulièrement intéressantes, ont été notamment obtenues en appliquant le procédé selon l'invention, les lignées correspondant à la PG $E_2$ portent les références 8E57 et 42-61-48, et des échantillons ont été déposés à la Collection Nationale de Culture de Microorganismes (CNCM) à l'Institut Pasteur à Paris, le 4 Novembre 1983 respectivement sous les numéros I-254 et I-255; la lignée correspondant à la PG $E_1$ porte la référence 36F20 et a été déposée à la CNCM le 17 Décembre 1984 sous le N° I-403.

Les hybridomes de l'invention permettent d'obtenir des anticorps monoclonaux particulièrement intéressants, de forte affinité pour la PG E contre laquelle ils sont dirigés et d'une grande spécificité, c'est-à-dire qu'ils ne donnent qu'une faible réaction croisée avec les autres PG E et les prostaglandines d'un autre type. Ces anticorps monoclonaux sont un autre objet de l'invention. Ils peuvent être présentés, en quantités relatives données et sous une forme convenable dans un coffret de dosage, des PG E. On peut adjoindre éventuellement des anticorps polyclonaux anti-PG $E_3$ tels que ceux obtenus comme décrit précédemment, aux anti-PG $E_1$ et PG $E_2$.

L'affinité des anticorps est étudiée en effectuant un essai radio-immunologique dit de compétition; on détermine l'importance relative de la fixation de PG E marquée au tritium et non marquée sur les anticorps monoclonaux à étudier pour des mélanges de ces PG E en quantités données; Pour cela, on fait incuber 12 heures à 4°C des quantités croissantes de PG E non marquée avec une dilution du surnageant de chaque puits dans lequel se développent des clones secréteurs, en présence de 10 pg de la même PG E mais tritiée; on calcule la dilution pour que 50% de la PG E tritiée se fixe sur les anticorps présents; les résultats obtenus sont représentés selon une courbe de Scatchard, d'où l'on déduit, de façon connue, la constante d'association de l'anticorps monoclonal à la PG E qui traduit l'affinité des anticorps pour leur antigène. La méthode de calcul de la constante d'association est décrite notamment dans Ann. N.Y. Acad. Sci.51, p.660-672 (1949).

La spécificité des anticorps est étudiée en effectuant le même type d'essai radioimmunologique de compétition lors de la fixation sur l'anticorps monoclonal anti-PG E à étudier, mais la compétition a lieu entre la PG E tritiée correspondant à l'anticorps préparé et d'autres prostaglandines.

Ces méthodes radioimmunologiques simples permettent de mettre en évidence parmi les hybridomes obtenus par le procédé de l'invention ceux qui secrètent des anticorps aux propriétés particulièrement avantageuses.

Les hybridomes selon l'invention produisent des quantité simportantes d'anticorps monoclonaux, faciles à isoler et purifier, ayant une forte affinité pour la PG E correspondante et nettement spécifiques de cette PG E, un autre apect de l'invention est l'application de ces anticorps à la mise en oeuvre de procédés de dosage immunologiques de chacune des PG E, en présence ou non d'autres prostaglandines. Dans le cas des mélanges, un avantage de l'invention est de pouvoir utiliser des anticorps en proportions relatives définies.

Pour ces dosages immunologiques, dont le principe est connu du spécialiste, on utilisera des coffrets de réactifs qui sont un autre objet de l'invention. Ils comportent une quantité donnée d'au moins un anticorps monoclonal anti-PG E de l'invention, sous forme lyophilisée ou toute autre forme convenant à sa conservation sans altération de son activité immunologique, une gamme étalon de PG E, une dose de PG E marquée, et un diluant physiologique. (tampon phosphate salin, par exemple). Lorsque le mélange à doser contient de la PG $E_3$, le réactif pourra comporter une quantité donnée d'anticorps polyclonal anti-PG $E_3$ obtenu en immunisant de manière classique, par des injections successives, des lapins avec la PG $E_3$, associée à de l'adjuvant complet de Freund (10 µg de PG $E_3$ par injection) le sérum polyclonal anti-PG $E_3$ ainsi obtenu titre au 1/2000.

10

Il est possible d'effectuer le dosage immunologique de la ou des PG E présentes dans un échantillon en utilisant de la PG E marquée par des radioéléments ou par tout autre moyen. Pour la description des procédés de dosage, on peut se référer aux articles de Dray F., Mamas S., Bizzini B. dans "Methods in Enzymology". Edt W. Lands & W. Smith 86 p 258 (1982) et de Dray F., dans le même ouvrage p 297.

Un autre objet de l'invention est l'application de ces anticorps spécifiques à la . modification in vitro ou in vivc des activités biologiques des PG E correspondants.

On a en effet montré que, par exemple, la présence d'antiPG $E_2$ selon l'invention empêchait la fixation de la PG $E_2$ à ses récepteurs spécifiques en étudiant la contraction du muscle du fundus de rat, par la méthode décrite par Vane J.R. dans Brit J. Pharmacol. Chematherap. 23 p. 360-373 (1964) ainsi que l'inhibition de la fixation de la PG $E_2$ aux nombreux récepteurs membranaires des cellules hypothalamiques du rat par la méthode décrite par MALET et COLL. dans Brain Rest.236 p. 227-233 (1982).

Les exemples de mise en oeuvre de l'invention qui suivent illustrent l'invention san pour autant la limiter.

EXEMPLE 1

Obtention des lignées d'hybridomes N° 42-61-48 et 8E57, secrétant des anticorps PG $E_2$

1) Immunisation des souris

De la PG $E_2$ (commercialisée par UPJOHN) est couplée à la thyroglobuline bovine en utilisant l'éthyl-1(diméthyl-amino-3 propyl)-3 carbodiimide, comme décrit par DRAY et coll. dans l'article précédemment cité

Le complexe PG E$_2$/thyroglobuline ainsi préparé est injecté par voie intrapéritonéale à un lot de 50 souris Balb/c, (commercialisées par IFFA-CREDO-France) à raison de 10 ug d'équivalent PG E$_2$, en émulsion dans l'adjuvant complet de Freund, pour chaque souris de 20 g. Une injection de rappel, par voie intrapéritonéale, est effectuée un mois plus tard avec 5 ug d'équivalent PG E$_2$ en émulsion dans le même volume d'adjuvant incomplet de Freund. Trois jours avant le prélèvement de la rate et l'isolement des splénocytes, une injection intrapéritonéale et une injection intraveineuse de 2,5 ug chacune d'équivalent PG E$_2$ en solution dans 100 ul de tampon phosphate sont effectuées.

2) Obtention des hybridomes

a) Préparation des splénocytes

On prélève et dilacère stérilement la rate des souris immunisées pour obtenir une suspension cellulaire. Celle-ci est lavée 3 fois par du RPMI-1640, en présence d'antibiotiques, avant d'être utilisée pour la fusion.

b) Fusion

On mélange dans des tubes les splénocytes à raison de 10$^7$ cellules par tube, et des cellules de myélome SP 2/0-Ag, à raison de 3 x 10$^6$ cellules par tube et on ajoute 1 ml de PEG-1000 commercialisé par MERCK dans chacun d'eux. Après 1 mn d'incubation à température ambiante, on ajoute 5 ml de RPMI-1640 en 3 minutes, puis 15 ml de RPMI-1640. On ajuste ensuite le volume à 50 ml avec du RPMI-1640, contenant 20% en volume de sérum cheval. On centrifuge alors pour éliminer le surnageant; les cellules décantées sont reprises dans le milieu de sélection.

c) Sélection

On distribue les cellules dans des puits de culture sur des microplaques à raison de $2 \times 10^5$ cellules environ par puits; on utilise pour cela des plaques de 24 puits LINBRO de Falcon remplis d'un milieu sélectif, composé de RPMI-1640 contenant 10% de sérum de cheval et d'

| | |
|---|---|
| azasérine | $10^{-5}$ M |
| hypoxanthine | $5 \times 10^{-5}$ M |
| glutamine | $2 \times 10^{-3}$ M |
| pyruvate de sodium | $10^{-3}$ M |
| pénicilline base | $5 \times 10^4$ UI/I |
| streptomycine | $5 \times 10^4$ UI/I |

Les hybridomes sont ainsi sélectionnées en 10 jours environ.

d) Criblage

On prélève les surnageants dans les puits où se sont développées de nombreuses cellules ($10^5$ environ par puits) et on les soumet aux essais radioimmunologiques précédemment décrits. Lorsque les surnageants des puits représentent une activité anti-PG $E_2$, on étudie l'affinité des anticorps présents par le test de liaison précédemment décrit. Lorsque la constante d'association est trouvée supérieure à $10^8$, on effectue le clonage et sous clonage des cellules du puits correspondant.

e) Clonage et sous-clonage
selon une technique connue, appelée dilution limite en milieu liquide

3) Caractérisation des hybridomes obtenus

On mesure l'affinité et la spécificité des anti-corps secrétés dans le surnageant, par les méthodes radio-immunologiques de compétition de liaison précédemment décrites.

On a trouvé quatre clones particulièrement intéressants, qui portent les références 42-61-48, 8E57, 38-200.10, 42-61-48.46 .

Les hybridomes de deux des lignées cellulaires (42-61-48 et 8E57) secrètent des anti- corps ayant une forte affinité pour la PG $E_2$, puisque la constante d'affinité est Ka=5 x $10^9$ $M^{-1}$ pour le premier et $10^{10}M^{-1}$ pour le second. A titre de comparaison, la constante d'affinité des meil- leurs anticorps polyclonaux de lapin préparés jusqu'à présent est de 2 à 3 $10^{10}M^{-1}$. En outre, ces hybridomes secrètent des quantités importantes d'anticorps puisque 1 ml d'une culture contenant $10^6$ cellules contient 5 µg environ d'anti-corps après 10 jours.Par ailleurs, lorsque ces hybridomes ont été injecté sà des souris Balb/c par voie intrapéritonéale, le liquide d'ascite formé conte- nait environ 800 ug/l d'anticorps.

La spécificité des anticorps secrétés par les hybridomes 42-61-48 est excellente. Ils sont de type IgG Cb Kapa. En effet, pour déplacer, dans les conditions de l'essai radio-immunologique de compétition définies précédemment, 2,5 pg de PG $E_2$ tritiée liée à l'anticorps à étudier, il faut 80 pg de PG $E_2$ non marquée, et 350 pg de PG $E_1$, 1000 pg de 6-céto PG $E_1$, 40 000 pg de PG $F_1$alpha, 500 000 pg de PG $B_2$ et 50 000 pg de PG $A_2$ ; ces résultats montrent que la réaction croisée avec la PG $E_1$ est faible, et celle avec la PG $B_2$ est ex- trêmement faible.

Pour la seconde lignée d'hybridomes, dans le même type d'essai, il faut 40 pg de PG $E_2$

non marquée, 70 pg de PG $E_1$, 350 pg de 6-céto-PG $E_1$, plus 500 pg de PG $A_2$ et plus de 10 000 pg de PG $B_2$ pour déplacer 2,5 pg de PG $E_2$ tritiée de sa liaison à l'anticorps. En ce qui concerne la souche 38-200.10, sa caractéristique principale est de présenter des réactions croisées moindre avec des produits tels que le 6-céto PG $E_1$. Sa constante d'affinité est de 9,1 x $10^9M^{-1}$. Cette souche a été déposée le 13 Décembre 1984 à la CNCM sous le N°I-401 Les anticorps produits sont de type IgG1 Kapa.

La souche 42-61-48.46 se distingue des 3 autres clones par une meilleure discrimination entre la PG $E_2$ et la PG $E_1$. Elle a été déposée le 13 Décembre 1984 à la CNCM sous le N° I-402 Sa constante d'affinité est 4,5 x $10^9M^{-1}$.

EXEMPLE 2

Obtention de la lignée d'hybrodomes N° 36-F-20 dirigée contre la PG $E_1$.

Parmi les animaux immunisés selon le procédé décrit dans l'exemple 1-1, certains (3/21) ont sécrété de façon surprenante des anticorps qui se complexaient plus fortement à la PG $E_1$ qu'à la PG $E_2$.

Les splénocytes de ces animaux ont été traités comme décrit à l'exemple 1, ce qui a conduit à l'isolement d'une lignée d'hybridomes secréteurs d'anticorps anti-PG $E_1$. Les anticorps produits par cette lignée sont de type IgG 1 Kapa. Leur constante d'affinité est 2 x $10^{10}M^{-1}$. Des échantillons de ces hybrodomes ont été déposés à la CNCM le 17 Décembre 1984 sous le N° I-403.

REVENDICATIONS

1. Anticorps dirigé contre une prostaglandine,caractérisé en ce qu'il est monoclonal et spécifique d'une PG E.

2 Anticorps monoclonal selon la revendica-tion 1, anti-PG $E_2$,caractérisé en ce qu'il est secrété par un hybridome ayant les caractéristiques de ceux déposés à la Collection Nationale de Cultures de Microorganismes (CNCM)de L'institut Pasteur sous le n°I-254.

3. Anticorps monoclonal selon la revedndi-cation 1 anti-PG $E_2$,caractérisé en ce qu'il est secrété par un hybridome ayant les caractéristiques de ceux déposés à la CNCM de l'Institut Pasteur sous le n°I-255 .

4. Anticorps monoclonal selon la revendi-cation 1 anti-PG $E_2$, caractérisé en ce qu'il est secrété par un hybridome ayant les caractéristiques de ceux déposés à la CNCM sous le n° I-401 .

5. Anticorps monoclonal selon la revendica-tion 1 anti-PG $E_2$,caractérisé en ce qu'il est secrété par un hybridome ayant les caractéristiques de ceux déposés  à la CNCM sous le n° I-402 .

6. Anticorps monoclonal selon la revendica-tion 1 anti-PG $E_1$,caractérisé en ce qu'il est secrété par un hybridome ayant les caractéristiques de ceux déposés à la CNCM sous le n° I-403 .

7. Composition d'anticorps monoclonaux se-lon la revendication 1,caractérisée en ce qu'elle contient en proportions définies des anticorps anti-PG $E_1$ spécifiques et anti-PG $E_2$ spécifiques.

8. Composition selon la revendication 7 contenant en outre des anticorps anti-PG $E_3$ .

9. Lignée cellulaire d'hybridomes, caractérisée en ce qu'elle secrète des anticorps monoclo-

16

0148075

naux spécifiques selon la revendication 1.

10. Lignée cellulaire d'hybridomes ayant les caractéristiques des échantillons déposés à la CNCM sous le N° I-254.

11. Lignée cellulaire d'hybrodomes ayant les caractéristiques des échantillons déposés à la CNCM sous le N° I-255.

12. Lignée cellulaire d'hybridomes ayant les caractéristiques des échantillons déposés à la CNCM sous le N°I-401

13. Lignée cellulaire d'hybrodomes ayant les caractéristiques des échantillons déposés à la CMCM sous le N° I-402.

14. lignée cellulaire d'hybrodomes ayant les caractéristiques des échantillons déposés à la CNCM sous le N° I-403

15. Procédé d'obtention d'hybrodomes sécreteurs d'anticorps monoclonaux selon l'une quelconque des revendications 1 à 6, caractérisé en ce que :

1) on effectue la fusion des cellules spléniques de souris, immunisées contre une PG E couplée à un agent immunogénique, avec des cellules de myélome murin,

2) on sélectionne les hybrodomes en étudiant les surnageants des clones par un test de compétition radio-immunologique.

16. Procédé de dosage immunologique d'une PG E caractérisé en ce que le réactif immunogénique comprend un anticorps monoclonal selon l'une quelconque des revendications 1 à 6.

17. Procédé de dosage immunologique d'un mélange de PG E caractérisé en ce que le réactif immunogénique comprend un mélange en proportions définies d'anticorps monoclonaux selon l'une quelconque des revendications 1 à 6.

18. Procédé de dosage immunologique d'un mélange de PG E comportant de la PG $E_3$,caractérisé en ce que le réactif immunogénique comprend un mélange en proportions définies d'au moins un anticorps monoclonal anti-PG $E_1$ ou anti-PG $E_2$ selon l'une des revendications 1 à 6 et d'anticorps polyclonaux anti-PG $E_3$.

19. Coffret de réactifs pour l'application d'un procédé selon l'une des revendications 16 et 17, caractérisé en ce qu'il comporte au moins un anticorps monoclonal selon l'une des revendications 1 à 6, de la PG E marquée, et une gamme étalon de PG E.

20. Coffret de réactifs pour l'application d'un procédé selon la revendication 18, caractérisé en ce qu'il comporte au moins un anticorps monoclonal anti PG $E_1$ ou anti PG $E_2$, un anticorps polyclonal anti-PG $E_3$, les PG E marquées et une gamme étalon.

21. Procédé de dosage immunologique d'un mélage de PG E, caractérisé en ce que le réactif immunogénique comprend une composition d'anticorps monoclonaux selon l'une des revendications 7 ou 8.

# DECLARATION SELON LA REGLE 28, PARAGRAPHE 4
# DE LA CONVENTION SUR LE BREVET EUROPEEN

Le demandeur a informé l'Office européen des brevets que, jusqu'à la publication de la mention de la délivrance du brevet européen ou jusqu'à la date à laquelle la demande est rejetée, retirée ou réputée retirée, l'accessibilité au(x) micro-organisme(s) identifié(s) ci-dessous, visée au paragraphe 3 de la règle 28 de la Convention sur le brevet européen, ne peut être réalisée que par la remise d'un échantillon à un expert.

## IDENTIFICATION DES MICRO-ORGANISMES

Numéros d'ordre des dépôts :

CNCM   —    I — 254
                            255
                            403
                            402
                            401